# EUROPEAN PATENT SPECIFICATION

(11) **EP 0 424 899 B1**
(45) Date of publication and mention of the grant of the patent: **22.01.1997**
(21) Application number: 90120373.7
(22) Date of filing: 24.10.1990
(51) Int. Cl.: C07H 15/252, A61K 31/71

(54) **Novel anthracycline glycoside derivatives and preparing method thereof**
Anthracyclinglycosid-Derivate und Verfahren zu ihrer Herstellung
Glycosides d'anthracyclines et procédé de leur préparation

(30) Priority: 25.10.1989 KR 1537589; 13.10.1990 KR 1621390
(43) Date of publication of application: 02.05.1991
(73) Proprietor: DONG-A PHARM. CO., LTD., Dongdaemoon-ku Seoul 130-070 (KR); ZAIDAN HOJIN BISEIBUTSU KAGAKU KENKYU KAI, Shinagawa-ku Tokyo (JP)
(72) Inventor: Kwang Dae, Ok, Suwon-shi, Kyounggi-do (KR); Jeong Bae, Park, Seoul (KR); Moon Sung, Kim, Seoul (KR)
(74) Representative: Kraus, Walter, Dr.

(56) References cited:
- EP-A- 0 104 654
- EP-A- 0 230 013
- EP-A- 0 275 431
- EP-A- 0 335 369
- FR-A- 2 135 221
- J. MED. CHEM. vol. 29, 1986, pages 1273-1276, American Chemical Society, M. ISRAEL et al.

## Description

### Field of the invention

The present invention relates to novel anthracycline glycoside derivatives of the following formula (1) 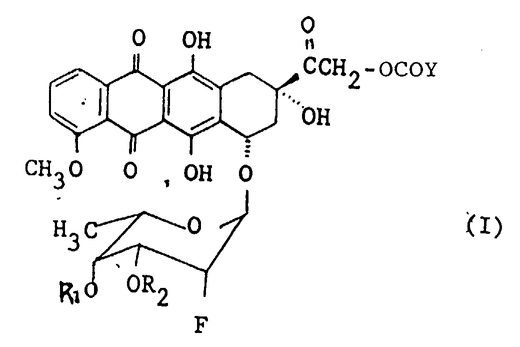 wherein R₁ and R₂ represent respectively hydrogen atom or together form straight or branched alkylidene group of 1-10 carbon atoms; Y represents 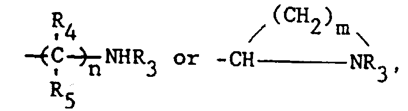 R₃ represents hydrogen atom, C₁₋₁₀ alkyl group or C₁₋₁₀ alkoxycarbonyl; R₄ and R₅ represent respectively hydrogen atom or C₁₋₅ alkyl group; n represents 0 or an integer of 1-10, and m represents an integer of 1-5 and pharmaceutically acceptable salt thereof.

### Description of the Prior Arts

As the antibiotics of anthracycline series, daunorubicin (USP 3,997,662) and doxorubicin (USP 3,590,028) were obtained from the fermented broth of Actinomyces species. The anthracyclines have broad spectrum antitumor activity and have been used as chemotherapeutics against malignant tumors.

The structural formula(A) of the above anthracyclines is as follows; 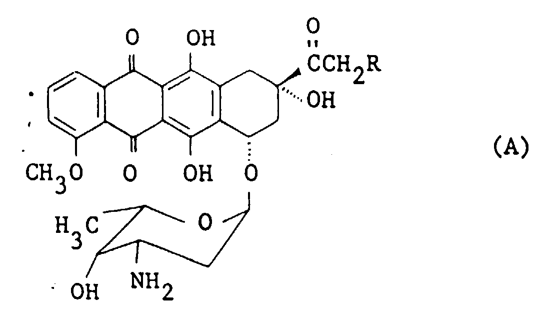 wherein R represents hydrogen atom or hydroxyl group.
In addition, 2-fluoro-substituted anthracycline derivative(B) was reported in Japanese Patent Laid Open Publication No. Sho 62-145,097. 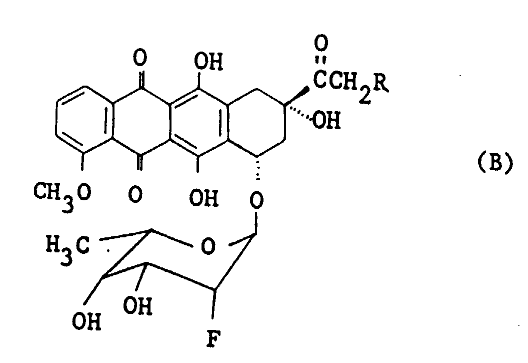 wherein R represents hydrogen atom or hydroxyl group.

Moreover, as a soluble derivative of the said compound(B), the compound(C) was reported in Japanese Patent Laid Open Publication No. Sho63-141,992. 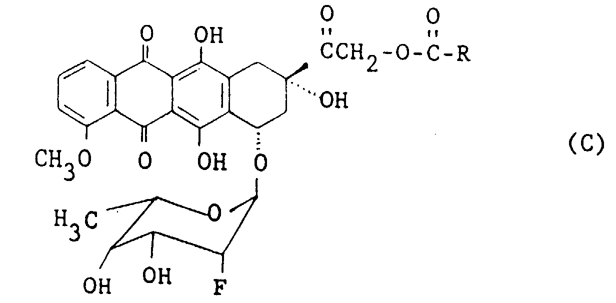 wherein R represents -(CH₂)ₘ H (m represents 0 or an integer of 1 to 6) or -(CH₂)ₙ COOH(n represents 0 or an integer of 1 to 10.) Certain undesirable side effects, however, have limited the usefulness of the above known anthracyclines such as daunorubicin and doxorubicin, etc. One of their more serious side effects is their cardiotoxicity which severely restricts the dosages and the frequency with which they can be administered and in turn, limits their overall effectiveness as a chemotherapeutic agent against malignant tumors.
And one of the other disadvantages of the above known compounds is that they have relatively low solubility in water.
In view of the low water solubility, these compounds are difficult to administer in amounts which would be effective in the treatment of some cancers.
The present inventors carried out an intensive study to solve such problems and found out surprisingly that the compound of formula(I) or its salts shows good antitumor activities with low toxicity and good solubility in isotonic water or even in neutral water, and accordingly completed the present invention. 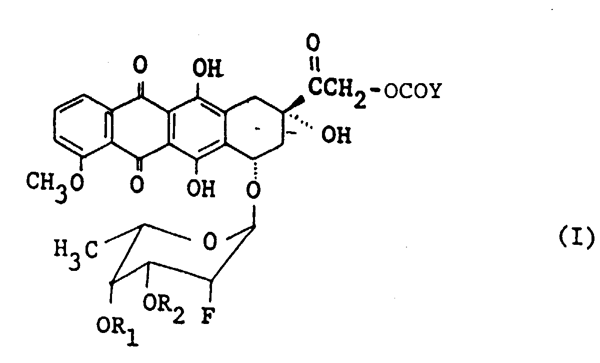 wherein R₁ and R₂ represent respectively hydrogen atom or together form straight or branched alkylidene group of 1-10 carbon atoms,
Y represents 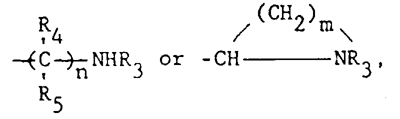 R₃ represents hydrogen atom, C₁₋₁₀ alkyl group or C₁₋₁₀ alkoxycarbonyl, R₄ and R₅ represent respectively hydrogen atom or C₁₋₅ alkyl group, n represents 0 or an integer of 1-10, and m represents an integer of 1-5, and pharmaceutically acceptable salt thereof.

### Summary of the Invention

Therefore, one object of the present invention is to provide a novel anthracycline derivative represented by the formula (I) and pharmaceutically acceptable salt thereof.

Another object of the present invention is to provide a process for the preparation of the anthracycline derivative represented by the formula(I) and pharmaceutically acceptable salts thereof.
The pharmaceutically acceptable salts mentioned in the present invention means inorganic acid addition salts such as halide, phosphate, sulfate, nitrate, and etc. ; organic acid addition salts such as acetate, methanesulfonate, benzenesulfonate and p-toluenesulfonate, etc. The title compound of the formula(I) or the salt thereof appears to display significant antitumor activity in animals with less toxicity than the other derivatives mentioned in the above.

The compound of the formula(I) is prepared by reacting a compound of the formula(II) in which tlhe hydroxyl groups in 3',4'-positions may be protected, 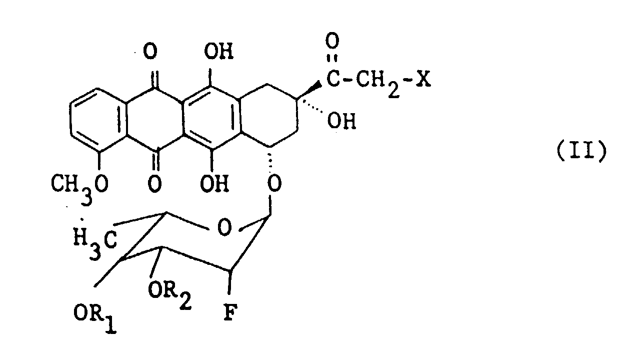 wherein R₁ and R₂ are the same as defined in the above and X represents bromine, chlorine or iodine atom, with a compound of the formula (III)

A-OCOY (III)

wherein Y is the same as defined in the above and A represents hydrogen atom or an alkali metal.
The protecting groups in the 3',4'-positions and amino protecting group are removed, and then the obtained compound is converted to the pharmaceutically acceptable acid addition salt, if necessary. The salt of the compound of the formula(I), however, can be easily prepared in the process of the above deprotecting reaction. For example, in the preparing process of the compound of the formula(I) or its salt, in case that X represents bromine and hydroxyl groups in 3',4'-positions are protected by isopropylidene, the reaction equation can be depicted as follows; 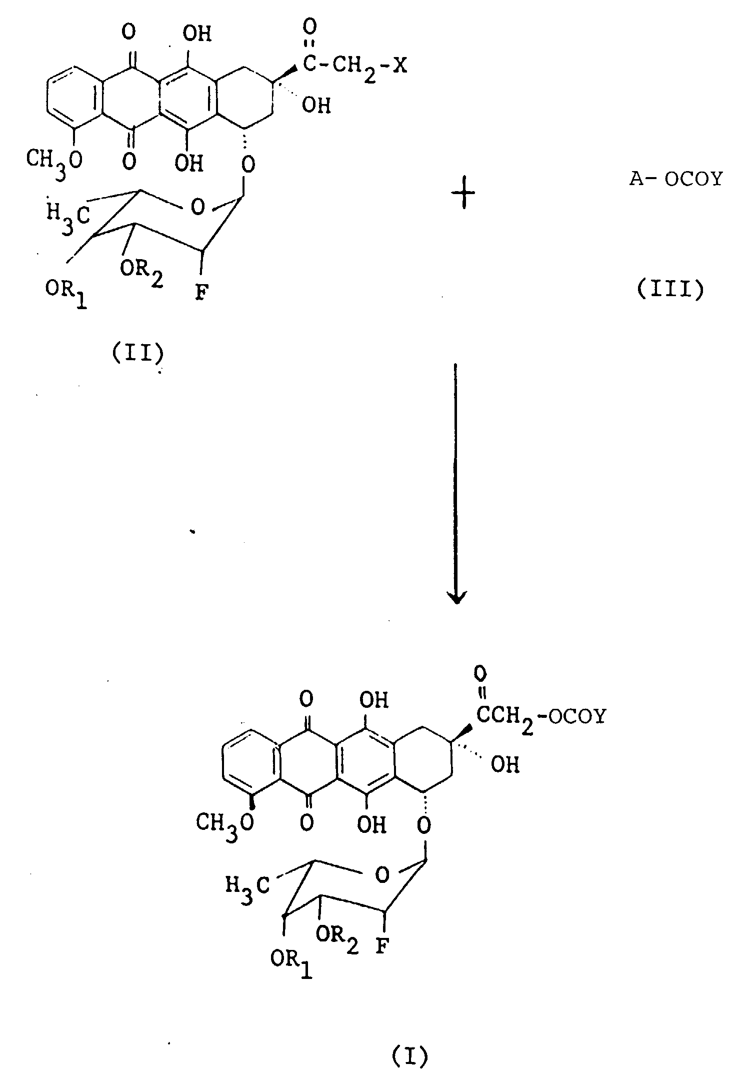 wherein R₁ ,R₂, Y and A are the same as defined in the above.

The reaction between the compound of the formula(II) and the compound of the formula(III) can be carried out in a conventional solvent such as, for example, water; alcohols, e.g. ethanol; nitriles, e.g. acetonitrile; ketones, e.g. acetone or methylethylketone; cyclic or aromatic amines, e.g. pyridine, pyrrolidine or pyrroline; aromatic hydrocarbones, e.g. benzene, toluene; ethers, e.g. dioxane, tetrahydrofurane; halogenated hydrocarbons, e.g. chloroform, dichloromethane; and amides e.g. formamide, dimethylformamide, dimethylacetamide or mixed solvents thereof. The reaction may be carried out between 0°C boiling point of the solvent used for 30 minutes-48 hours.

In carrying out the present reaction, hydroxyl groups can be protected before the reaction and the protected hydroxyl groups can be deprotected after the reaction. As a protecting group, straight or branched alkylene group can be used.

The starting compound of the formula(II) of the present invention can be obtained from the compound described in the Japanese Patent Laid Open Publication No. Sho62-145097.

If it is required that the hydroxyl groups of the compound of the formula(I) are deprotected, the protected compound is deprotected with acid such as formic acid, acetic acid, hydrochloric acid, sulfuric acid or phosphoric acid, The amino protecting group can be easily removed, too.

Solvent which may be used in the deprotecting reaction is non-protonic solvent such as water, alcohol, DMF, DMSO, dioxane, ether, chloroform, THF, dichloromethane or the mixtures thereof. The deprotecting reaction can be carried out between 0°C-boiling point of solvent used in the reaction. If necessary, the compound of the formula(I) may be converted to the salt thereof in a conventional organic solvent such as alcohol, dichloromethane, ether, chloroform, THF, or dioxane.

The present invention is explained in more detail by the examples.

### Example 1

### Preparation of 7-0-(2,6-dideoxy-2-fluoro-3,4-0-isopropylidene-α-L-talopyranosyl)-adriamycinone 14-0-[N-(t-butyloxycarbonyl(Boc))-glycinate]

To a solution of 14-bromo-7-0-(2,6-dideoxy-2-fluoro-3,4-0-isopropylidene-α-L-talopyranosyl)daunomycinone (180mg) in aqueous aceton (7:26, 33ml) was added sodium N-t-butyloxycabonyl-glycinate(1.5g). The mixture was stirred for 20 hours and was distilled under reduced pressure to obtain a residue. The residue was extracted by chloroform washed with water and saturated NaCl solution successively and was dried in vacuo. The residue was purified by silicagel chromatography(eluting solvent: mixed solvent of chloroform :methanol=20:1) to obtain 121mg of the title compound(59%).
MP: 142-143.5°C
NMR(CDCl₃ , ppm, specific peak)
13.7, 13.1(OH x 2)
5.53(d.d, 1H, H-1′, J_{H-1′-H-2′} -5.6Hz,
³J_{H-1′-F} =14Hz),
4.01(s, 3H, OCH₃ ),
1.55(s, 3H, CH₃ ),
1.32(s, 3H, CH₃ ),
1.30(d, 3H, CH₃ J_{CH₃-H-5′} =6.4Hz),
1.44(s, 9H, t-butyl).

### Example 2

### Preparation of 7-0-(2,6-dideoxy-2-fluoro-α-L-talopyranosyl)-adriamycinone 14-0-glycinate HCl salt

The compound(100mg) obtained in the Example 1 was dissolved in 80% acetic acid solution(10ml) and was stirred at 80°C for 3 hours. Solvent was distilled under reduced pressure to obtain a residue. The residue was chromatographed on a column of silica gel (eluting solvent; chloroform:methanol-5:1) to obtain 7-0-(2,6-dideoxy-2-fluoro-α-L-talopyranosyl)-adriamycinone 14-0-glycinate. This compound was dissolved in small amount of dichloromethane. Saturated HCl-ether solution was dropwise added to the solution to obtain a red solid. The obtained a red solid was washed with ether, centrifuged and dried to obtain the title compound as HCl salt (48mg, 56%).
MP : 179-185°C
NMR(CDCl₃, ppm, specific peak)
14.0, 13.2(s, each 1H, OH x 2),
8.3 (hrs, 3H, -NH₃Cl),
7.9 (m, 2H, aromatic proton),
7.6 (m, 1H, aromatic proton),
4.0 (m, 5H, OCH₃, CO-CH₂-N),
1.2 (d, 3H, CH₃, J_{CH₃-H₅′} = 6.4Hz).

### Example 3

### Preparation of 7-0-(2,6-dideoxy-2 fluoro-α-L-talopyranosyl)-adriamycinone 14-0-glycinate HCl salt

The compound(120mg) obtained in the Example 1 was dissolved in chloroform(1.5mℓ ) and metanol(15mℓ) was added thereto. Saturated HCl-ether solution(12mℓ) was added thereto and the mixture was stirred for 4 hours. After the completion of reaction, a considerable amount of solvent was distilled to obtain a residue. Ether was added to the residue to obtain a solid.
This solid was filtered and dried to obtain the title compound (84mg. 80%).

### Example 4

### preparation of 7-0-(2,6-dideoxy-2-fluoro-3,4-0-isopropylidene-α-L-talopyranosyl)-adriamycinone 14-0- [N-(t-butyloxycarbonyl(Boc))-β-alaninate]

14-bromo-7-0-(2,6-dideoxy-2-fluoro-3,4-isopropylidene-α-L-talopyranosyl)daunoycinone(200mg) was reacted with sodium N-(t-butyloxycarbonyl)-β-alaninate (634mg) by the analogous method of Example 1 to obtain the title compound (144mg, 62%) as a red solid. Mixture solvent of benzene and acetone(4:1) was used as eluting solvent for silica gel chromatography.
MP : 138-140.5°C
NMR(CDCl₃, ppm, specific peak)
13.8, 13.2(s, each 1H, OH x 2),
5.5(d.d, 1H, H-1′, J_{H-1′-H-2′}=5.6Hz, J_{H-1′-F}=13.8Hz),
4.1(s, 3H, OCH₃),
1.5, 1.4 (s, each 3H, CH₃x 2)
1.3(d, 3H, CH₃, J_{CH₃-H-5′}= 6.4Hz),
1.4(s, 9H, t-butyl).

### Example 5

### Preparation of 7-0-(2,6-dideoxy-2-fluoro-α-L-talopyranosyl)-adriamycinone 14-0-β-alaninate HCl salt

The compound (140mg) obtained in Example 4 was treated by the analogous method of Example 3 to obtain the title compound (92mg, 76%).
MP : 195-200°C.
NMR(DMSO-d₆ , ppm, specific peak)
14.0, 13.2(s, each 1H, OH x 2),
7.9(m, 5H, -NH₃Cl, 2H(aromatic proton)),
7.6(m, 1H, 1H(aromatic proton)),
3.9(s, 3H, OCH₃ ),
1.1(d, 3H, CH₃ , J_{CH₃-H-5′} =6.5Hz).

### Example 6

### Preparation of 7-0-(2,6-dideoxy-3,4-0-isopropylidene-α-L-talopyranosyl)-adriamycinone 14-0-[6-(t-butyloxycarbonyl(Boc))-amino hexanoate]

14-bromo-7-0-(2,6-dideoxy-2-fluoro-3,4-0-isopropylidene-α-L-talopyranosyl)daunomycinone(220mg) was reacted with sodium 6-(t-butyloxycarbonylamino)hexanoate(900mg) by the analogous method of Example 1 to obtain the title compound(158mg, 64%) as a red solid. Mixed solvent of benzene and acetone(4:1) was used as eluting solvent for silicagel chromatography.
MP : 130-132°C
NMR(CDCl₃ ,ppm, specific peak)
13.8, 13.2(s,each 1H, OH x 2),
5.5(d.d, 1H, H-1′, J_{H-1′-H-2′}=5.5Hz, J_{H-1′-F}=13.8Hz),
4.0(s, 3H, OCH₃ ),
1.5(s, 9H, t-butyl),
1.6, 1.5(s, each 3H, CH₃ x 2),
1.3(d, 3H, CH₃ , J_{CH₃-H-5′} =6.4Hz),

### Example 7

### Preparation of 7-0-(2,6-dideoxy-2-fluoro-α-L-talopyranosyl)-adriamycinone 14-0-(6-aminohexanoate) HCl salt

The compound(150mg) obtained in Example 6 was treated by the analogous method of Example 3 to obtain the title compound(95mg, 72%).
MP : 188-192°C
NMR( DMSO-d₆, ppm, specific peak)
14.0, 13.2 (s, each 1H, OH x 2),
7.8(m, 3H, aromatic proton and amino),
7.6(m, 3H, aromatic proton and amino),
3.9(s, 3H OCH₃),
1.3(s, 6H, -CH₂-CH₂-CH₂-),
1.2(d, 3H, CH₃, J_{CH₃-H₅′} = 6.4Hz )

### Example 8

### Preparation of 7-0-(2,6-dideoxy-2-fluoro-3,4-0-isopropylidene-α-L-talopyranosyl)-adriamycinone 14-0- [N-(t-butyloxycarbonyl)-L-alaninate]

14-bromo-7-0-(2,6-dideoxy-2-fluoro-3,4-0-isopropylidene-L-talopyranosyl)daunoycinone(200mg) was reacted with sodium N-(t-butyloxycarbonyl)-L-alaninate(650mg) by the analogous method of Example 1 to obtain the title compound (137mg, 59%) as a red solid. Mixture solvent of benzene and acetone(4:1) was used as eluting solvent for silicagel chromatography.
MP : 148.5-151.0°C
NMR(CDCl₃ ,ppm, specific peak)
13.8, 13.2(s,each 1H, OH x 2),
5.5(d.d, 1H, H-1′, J_{H-1′-H-2′}=5.6Hz, J_{H-1′-F}=13.8Hz),
4.1(s, 3H, OCH₃ ),
1.5, 1.4(s, each 3H, CH x 2),
1.3(d, 3H, CH₃ , J_{CH₃-H-5′}=6.5Hz),
1.5(d, 3H, CH₃ , J_{CH₃-CH} =7.1Hz),
1.4(s, 9H, t-butyl).

### Example 9

### Preparation of 7-0-(2,6-dideoxy-2-fluoro-α-L-talopyranosyl)-adriamycinone 14-0-L-alaninate HCl salt

The compound(130mg) obtained in Example 8 was treated by the analogous method of Example 3 to obtain the title compound (90mg, 80%)
MP : 177-184°C
NMR(DMSO-d₆,ppm, specific peak)
14.0, 13.2(s, each 1H, OH x 2),
8.5(br, s, 3H, -NH₃Cl),
7.9(m, 2H, aromatic proton),
7.6(m, 1H, aromatic proton),
3.9(s, 3H, OCH₃),
1.5(d, 3H, CH₃, J_{CH₃-CH} =7.1Hz),
1.3(d, 3H, CH₃, J_{CH₃-H-5′} =6.5Hz).

### Example 10

### Preparation of 7-0-(2,6-dideoxy-2-fluoro-3,4-0-iospropylidene-α-L-talopyranosyl)-adriamycinone 14-0-[N-(t-butyloxycarbonyl)-L-valinate]

14-bromo-7-0-(2,6-dideoxy-2-fluoro-3,4-0-isopropylidene-α-L-talopyranosyl)daunomycinone(200mg) was reacted with sodium N-(t-butyloxycarbonyl)-L-valinate(700mg) by the analogous method of Example 1 to obtain the title compound(145mg, 60%) as a red solid. Mixture solvent of benzene and acetone(4:1) was used as eluting solvent for the silicagel chromatography.
MP : 137-139°C
NMR(CDCl , ppm, specific peak)
13.9, 13.2(s, each 1H, OH x 2),
5.5(d., 1H, H-1′, J_{H-1′-H-2′} =5.5Hz, J_{H-1′-F} =13.8Hz),
4.0(s, 3H, OCH₃),
1.4(s, 9H, t-butyl),
1.3(d, 3H, CH₃, J_{CH₃-H-5′} =6.4Hz),
1.0(d.d, 6H, -CH(CH₃)₂).

### Example 11

### Preparation of 7-0-(2,6-dideoxy-2-fluoro-α-L-talopyranosyl)-adriamycinone 14-0-L-valinate HCl salt

The compound(140mg) obtained in Example 10 was treated by the analogous method of Example 3 to obtain the title compound(90mg, 74%) as a red solid.
MP : 164-168°C
NMR(DMSO-d₆, ppm, specific peak)
14.0, 13.2(s, each 1H, OH x 2),
8.4(br.s., 3H, NH₃Cl),
7.9(m, 2H, aromatic proton),
7.6(m, 1H, aromatic proton),
4.0(s, 3H, OCH₃),
1.2(d, 3H, CH₃, J_{CH₃-H-5′} =6.5Hz),
1.0(d.d, 6H, -CH(CH₃)₂).

### Example 12

### Preparation of 7-0-(2,6-dideoxy-2-fluoro-3,4-0-isopropylidene-α-L-talopyranosyl)-adriamycinone 14-0-[N-(t-butyloxycarbonyl)-L-prolinate]

14-bromo-7-0-(2,6-dideoxy-2-fluoro-3,4-0-isopropylidene-α-L-talopyranosyl)daunomycinone(200mg) and sodium N-(t-butyloxycarbonyl)-L-prolinate(900mg) were treated by the analogous method of Example 1 to obtain the title compound(150mg, 62%) as a red solid. Mixed solvent of benzene and acetone(4:1) was used as eluting solvent for the silicagel chromatography.
MP : 145.5-148.5°C
NMR(CDCl₃, ppm, specific peak)
13.8, 13.2(s, each 1H, OH x 2),
5.5(d.d, 1H, H-1′, J_{H-1′-H-2′} =5.5Hz, J_{H-1′-F} = 13.8Hz),
4.0(s, 3H, OCH₃),
1.4(s, 9H, t-butyl),

### Example 13

### Preparation of 7-0-(2,6-dideoxy-2-fluoro-α-L-talopyranosyl)-adriamycinone 14-0-L-prolinate HCl salt

The compound(140mg) obtained in the Example 12 was treated by the analogous method of Example 9 to obtain the title compound (94mg, 77%) as a red solid.
MP : 180-185°C
NMR(DMSO-d₆, ppm, specific peak)
14.0, 13.1(s, each 1H, OH x 2),
10.2, 9.1(2 br.s., each 1H, -NH₃Cl),
7.9(m, 2H, aromatic proton),
7.6(m, 1H, aromatic proton),
3.9(s, 3H, OCH₃),
1.2(d, 3H, CH₃, J_{CH₃-H-5′} =6.3Hz).

### Biologic activity

### Experiment 1

The in vivo antitumor activity of the compounds of the present invention was studied on CDF₁ mice bearing L1210 murine leukemia. Healthy female CDF₁ mice were inoculated by intraperitoneal injection with 1 x 10⁵ L1210 leukemia cells per animal. The inoculated mice were then treated intraperitoneally on the days 1, 5, 9 beginning on day 1, 24hours after inoculation of the leukemia cells with various doses of test compounds. Doxorubicin hydrochloride was administered for comparison. All test compounds and doxorubicin hydrochloride were dissolved in filtered(0.22µm)distilled water. The animals were observed for 60 days after inoculation of the leukemia cells and their survivals were compared with that of control animals which received the same tumor inoculation but were treated with filtered physiological saline. The results are shown in Table 1, where T/C(%) values are determined by dividing the mean survival time of the treated mice by that of the control mice, the quotient so obtained being multiflied by 100. The survival time of the mice which survived more than 60 days after tumor inoculation was scored as 60 days. An increase on the T/C(%) indicates an increase in the antitumor activity of the compound.

The compounds of present invention proved to be less toxic than doxorubicin hydrochloride in that doxorubicin hydrochloride showed significant decrease in T/C(%) at the dose of 16mg/kg compared with T/C(%) at the dose of 8mg/kg, whereas the compounds of present invention showed no such toxicity. As shown in Table 1, most of the compounds of present invention turned out to have superior antitumor activity to that of doxorubicin hydrochloride. Moreover, the compound of Example 8 cured all tumored mice at the doses of 32mg/kg, 16mg/kg and 8mg/kg.

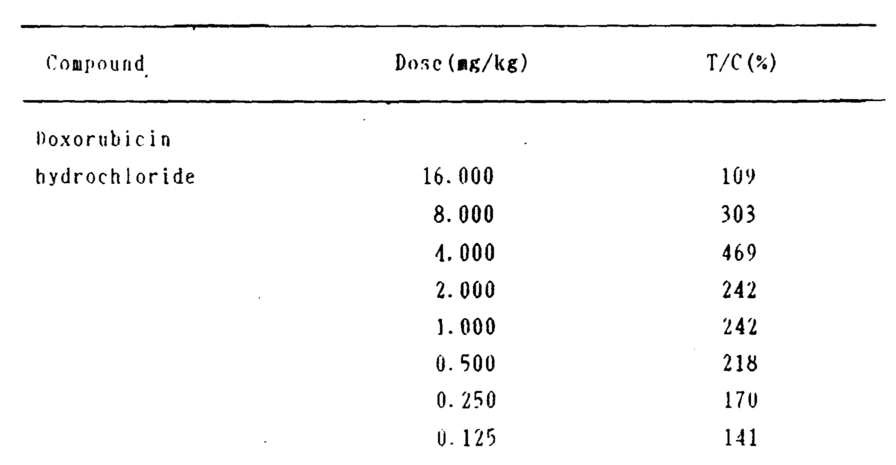

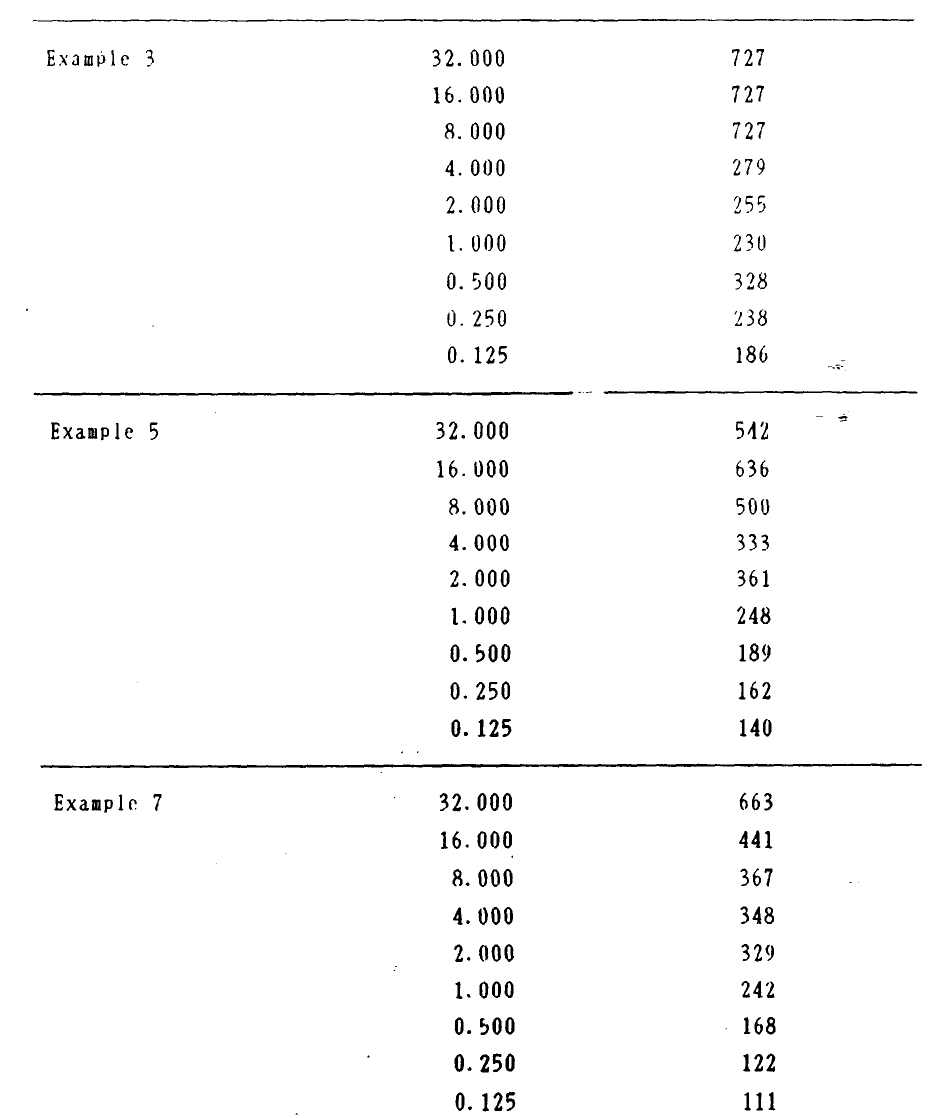

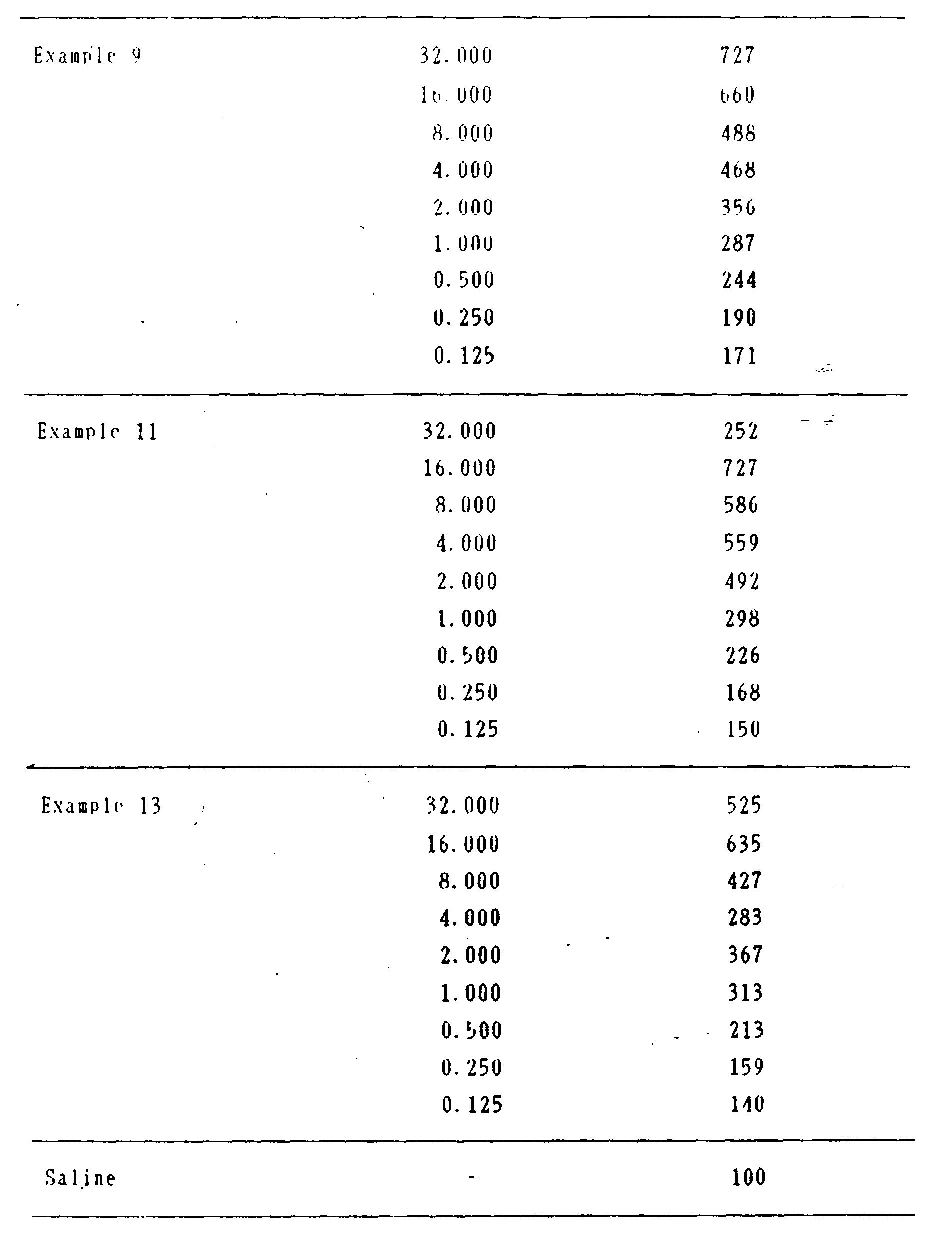

### Experiment 2

In vitro growth inhibition activity of the compounds of present invention on L1210 murine leukemia cells was assessed by tetrazolium-based colorimetric assay described in Cancer Research, Vol. 47, pp. 936-942, February 1987 with some modifications.

L1210 murine leukemia cells in exponential phase were plated at the density of 1 x 10⁵ cells per well in 96-well microliter plates. The cell culture medium was RPMI 1640(Roswell Park Memorial Institutes 1640) medium supplemented with 10% heat inactivated fetal bovine serum (FBS), 2mM L-glutamine, penicillin G sodium(100 units per ml of medium), and streptomycin sulfate(1mg per ml of medium).
The anthracycline compounds of present invention and doxorubicin hydrochloride were added to give final concentrations from 1ng per ml to 300ng per ml, while the control contained no drug.

The cells were incubated for 72 hours at 37°C in an atmosphere of 10% CO and 90% air. At the end of drug exposure, 50 µl MTT(3-(4,5-dimethylthiazol-2-yl)-2,5-diphenyltetrazolium bromide)solution(2mg per ml in phosphate-buffered saline) was added to each well and cultures were incubated at 37°C for 4 hours in CO₂ incubator. The MTT is reduced to water insoluble MTT formazan(1-(4,5-dimethylthiazol-2-yl)-3,5-diphenylformazan)by mitochondrial succinate dehydrogenase only in living cells. At the end of incubation, 200 µl of supernatant was removed and the MTT formazan crystal was dissolved by adding 200 µl dimethylsulfoxide with mixing them.

The plates were then read immediately at 540 nm on a scanning multiwell spectrophotometer(enzyme-linked immunosorbent assay reader; Biotech Instruments Inc., Burlington, VT).
The absorbance value corrected against uninoculated blank values. The absorbance values are directly proportional to the cell numbers in living state. The IC 50 defined at 50% reduction of absorbance against untreated control were calculated from dose-response curve and are shown in Table 2.

As shown in Talbe 2., all test compounds of present invention but the compound of example 7 turned out to be more cytotoxic than doxorubicin hydrochloride against L1210 murine leukemia cells.

**Table 2.**

| In vitro cytotoxicity of the compounds of present invention on L1210 murine leukemia cells. | |
|---|---|
| Compound | IC50(ng/ml) |
| Doxorubicin hydrochloride | 37.3 |
| Example 3 | 13.4 |
| Example 5 | 10.1 |
| Example 7 | 74.5 |
| Example 9 | 14.3 |
| Example 11 | 15.5 |
| Example 13 | 16.3 |

## Claims (Claims for the following Contracting State(s): AT, BE, CH, DE, DK, FR, GB, IT, LI, LU, NL, SE)

1. A compound of the formula (I) 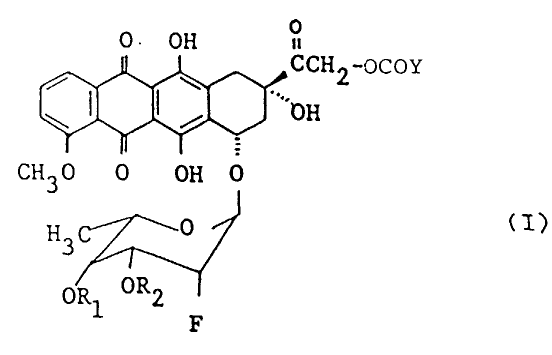 wherein R₁ and R₂ represent respectively hydrogen atom or together form straight or branched alkylidene group of 1-10 carbon atoms;
Y represents 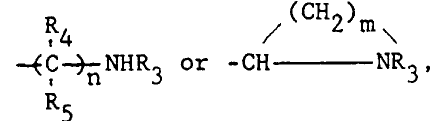 R₃ represents hydrogen atom, C₁₋₁₀ alkyl group or C₁₋₁₀ alkoxycarbonyl, R₄ and R₅ represent respectively hydrogen atom or C₁₋₅ alkyl group, n represents 0 or an integer of 1-10, and m represents an integer of 1-5, and pharmaceutically acceptable salt thereof.

2. A process for the preparation of a compound of the formula (I) 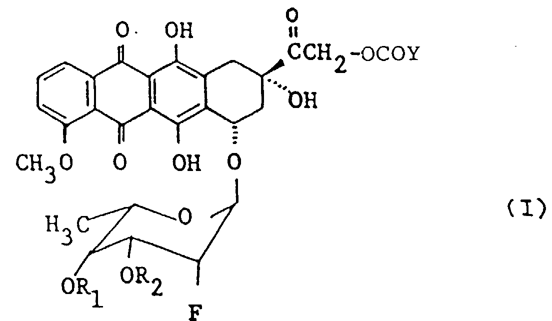 wherein R₁ and R₂ represent respectively hydrogen atom or together form straight or branched alkylidene group of 1-10 carbon atoms;
Y represents 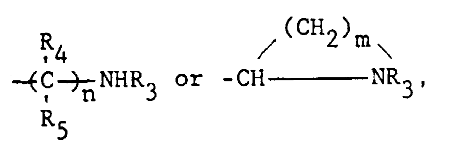 R₃ represents hydrogen atom, C₁₋₁₀ alkyl group of C₁₋₁₀ alkoxycarbonyl, R₄ and R₅ represent respectivley hydrogen atom or C₁₋₅ alkyl group, n represents 0 or an integer of 1-10, and m represents an integer of 1-5, and pharmaceutically acceptable salt thereof, by reacting a compound represented by the formula (II) 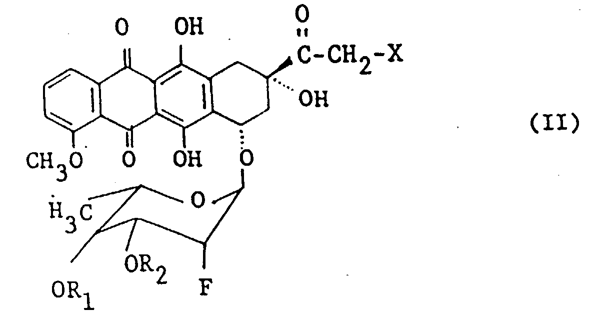 wherein R₁ and R₂ are the same as defined above; and X represents bromine, chlorine or iodine atom,
with a compound of the formula (III)
A-OCOY (III)
wherein Y is the same as defined above; and A represents hydrogen atom or an alkali metal,
or pharmaceutically acceptable salt thereof to obtain the compound of the formula (I) and, if necessary, the compound of the formula (I) is converted to the pharmaceutically acceptable salt thereof by a conventional way.

## Claims (Claims for the following Contracting State(s): ES)

1. A process for the preparation of a compound of the formula (I) 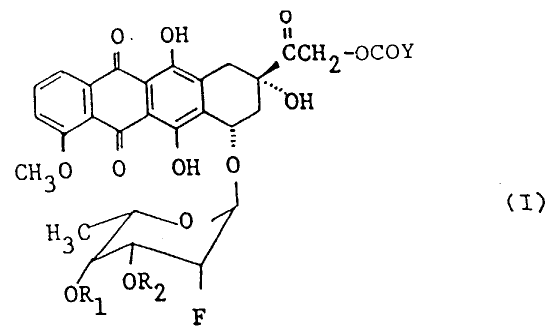 wherein R₁ and R₂ represent respectively hydrogen atom or together form straight or branched alkylidene group of 1-10 carbon atoms;
Y represents 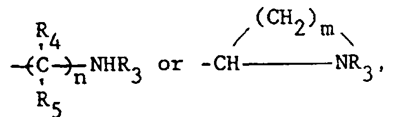 R₃ represents hydrogen atom, C₁₋₁₀ alkyl group or C₁₋₁₀ alkoxycarbonyl, R₄ and R₅ represent respectively hydrogen atom or C₁₋₅ alkyl group, n represents 0 or an integer of 1-10, and m represents an integer of 1-5, and pharmaceutically acceptable salt thereof, by reacting a compound represented by the formula (II) 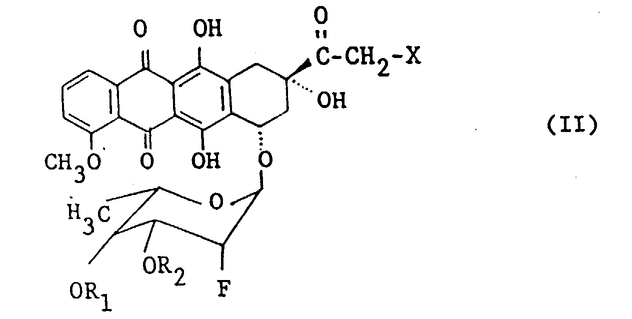 wherein R₁ and R₂ are the same as defined above; and X represents bromine, chlorine or iodine atom,
with a compound of the formula (III)
A-OCOY (III)
wherein Y is the same as defined above; and A represents hydrogen atom or an alkali metal,
or pharmaceutically acceptable salt thereof to obtain the compound of the formula (I) and, if necessary, the compound of the formula (I) is converted to the pharmaceutically acceptable salt thereof by a conventional way.

## Claims (Claims for the following Contracting State(s): GR)

1. A compound of the formula (I) 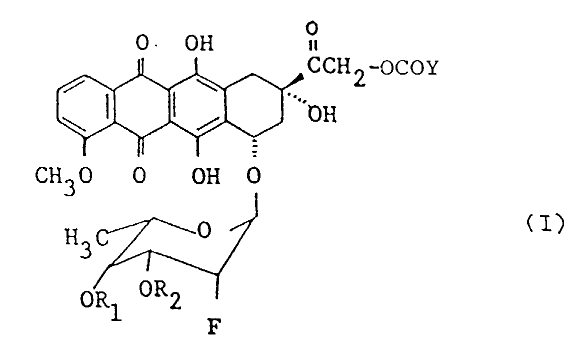 wherein R₁ and R₂ represent respectively hydrogen atom or together form straight or branched alkylidene group of 1-10 carbon atoms;
Y represents 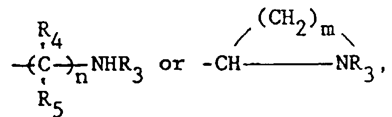 R₃ represents hydrogen atom, C₁₋₁₀ alkyl group or C₁₋₁₀ alkoxycarbonyl, R₄ and R₅ represent respectively hydrogen atom or C₁₋₅ alkyl group, n represents 0 or an integer of 1-10, and m represents an integer of 1-5, and salt thereof.

2. A process for the preparation of a compound of the formula (I) 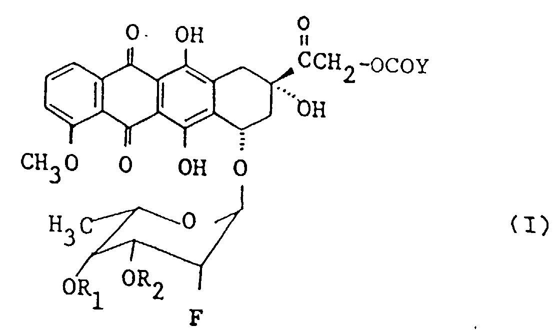 wherein R₁ and R₂ represent respectively hydrogen atom or together form straight or branched alkylidene group of 1-10 carbon atoms;
Y represents 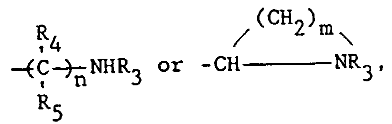 R₃ represents hydrogen atom, C₁₋₁₀ alkyl group of C₁₋₁₀ alkoxycarbonyl, R₄ and R₅ represent respectivley hydrogen atom or C₁₋₅ alkyl group, n represents 0 or an integer of 1-10, and m represents an integer of 1-5, and salt thereof, by reacting a compound represented by the formula (II) 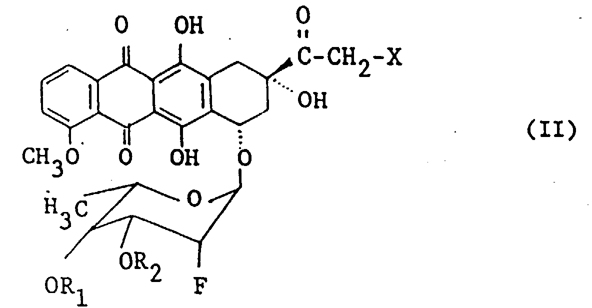 wherein R₁ and R₂ are the same as defined above; and X represents bromine, chlorine or iodine atom,
with a compound of the formula (III)
A-OCOY (III)
wherein Y is the same as defined above; and A represents hydrogen atom or an alkali metal,
or salt thereof to obtain the compound of the formula (I) and, if necessary, the compound of the formula (I) is converted to the salt thereof by a conventional way.

## Patentansprüche (Patentansprüche für folgende(n) Vertragsstaat(en): AT, BE, CH, DE, DK, FR, GB, IT, LI, LU, NL, SE)

1. Verbindung der Formel (I): 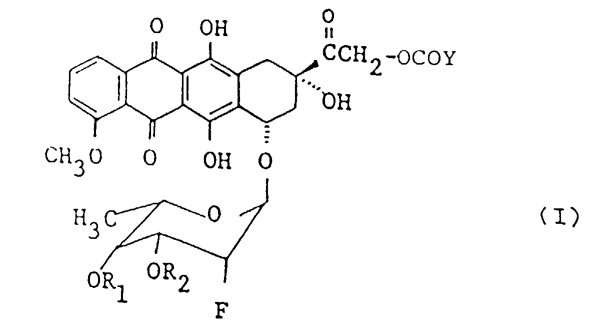 worin R₁ und R₂ ein Wasserstoffatom bedeuten oder zusammen eine geradkettige oder verzweigtkettige Alkylidengruppe mit 1 bis 10 Kohlenstoffatomen bilden,
Y 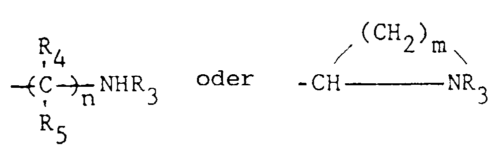 bedeutet,
R₃ ein Wasserstoffatom, eine C₁₋₁₀-Alkylgruppe oder eine C₁₋₁₀-Alkoxycarbonylgruppe bedeutet; R₄ und R₅ ein Wasserstoffatom oder eine C₁₋₅-Alkylgruppe bedeuten, n 0 oder eine ganze Zahl von 1 bis 10 bedeutet und m eine ganze Zahl von 1 bis 5 bedeutet,
und die pharmazeutisch annehmbaren Salze davon.

2. Verfahren zur Herstellung einer Verbindung der Formel (I): 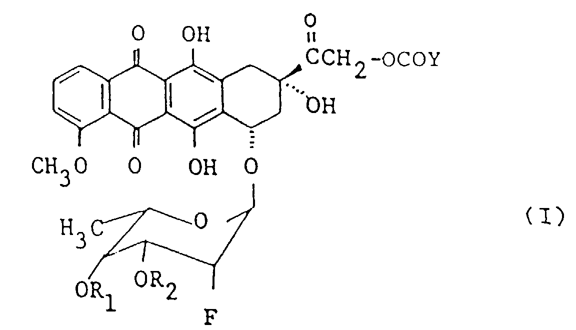 worin R₁ und R₂ ein Wasserstoffatom bedeuten oder zusammen eine geradkettige oder verzweigtkettige Alkylidengruppe mit 1 bis 10 Kohlenstoffatomen bilden,
Y 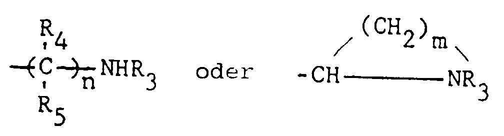 bedeutet,
R₃ ein Wasserstoffatom, eine C₁₋₁₀-Alkylqruppe oder eine C₁₋₁₀-Alkoxycarbonylgruppe bedeutet; R₄ und R₅ ein Wasserstoffatom oder eine C₁₋₅-Alkylgruppe bedeuten, n 0 oder eine ganze Zahl von 1 bis 10 bedeutet und m eine ganze Zahl von 1 bis 5 bedeutet,
und der pharmazeutisch annehmbaren Salze davon durch Umsetzung einer Verbindung der Formel (II): 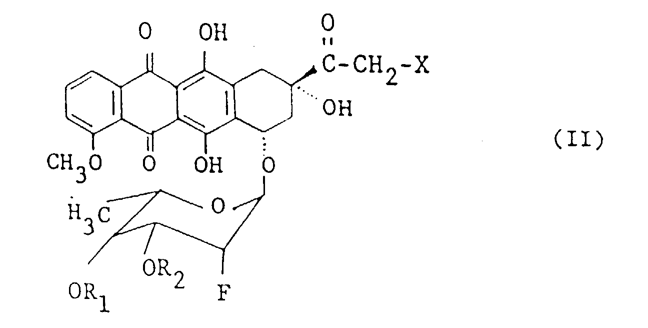 worin R₁ und R₂ die gleichen Bedeutungen, wie oben gegeben, besitzen und X ein Brom-, Chlor- oder Iodatom bedeutet,
mit einer Verbindung der Formel (III):
A-OCOY (III)
worin Y die gleiche Bedeutung, wie oben gegeben, besitzt und A ein Wasserstoffatom oder ein Alkalimetall bedeutet,
oder den pharmazeutisch annehmbaren Salzen davon unter Bildung einer Verbindung der Formel (I) und, wenn erforderlich, Umwandlung der Verbindung der Formel (I) in ein pharmazeutisch annehmbares Salz davon in an sich bekannter Weise.

## Patentansprüche (Patentansprüche für folgende(n) Vertragsstaat(en): ES)

1. Verfahren zur Herstellung einer Verbindung der Formel (I): 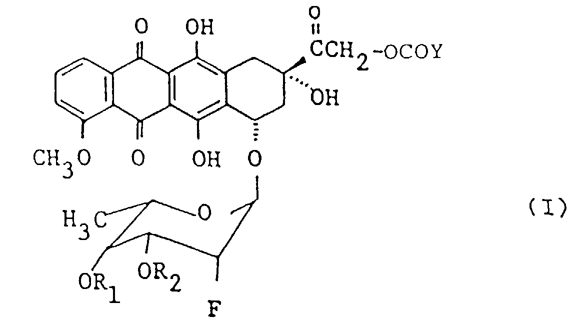 worin R₁ und R₂ ein Wasserstoffatom bedeuten oder zusammen eine geradkettige oder verzweigtkettige Alkylidengruppe mit 1 bis 10 Kohlenstoffatomen bilden,
Y 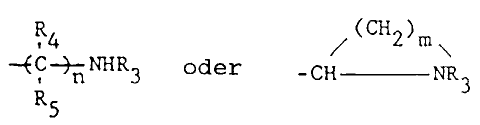 bedeutet,
R₃ ein Wasserstoffatom, eine C₁₋₁₀-Alkylgruppe oder eine C₁₋₁₀-Alkoxycarbonylgruppe bedeutet; R₄ und R₅ ein Wasserstoffatom oder eine C₁₋₅-Alkylgruppe bedeuten, n 0 oder eine ganze Zahl von 1 bis 10 bedeutet und m eine qanze Zahl von 1 bis 5 bedeutet,
und der pharmazeutisch annehmbaren Salze davon durch Umsetzung einer Verbindung der Formel (II): 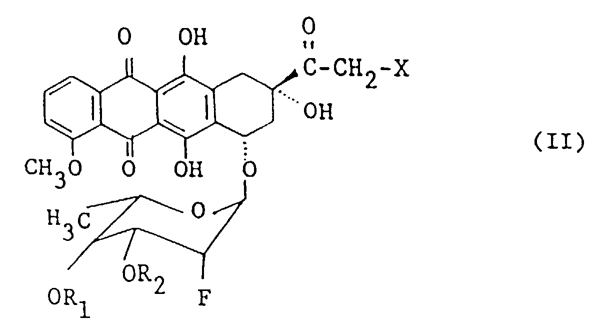 worin R₁ und R₂ die gleichen Bedeutungen, wie oben gegeben, besitzen und X ein Brom-, Chlor- oder Iodatom bedeutet,
mit einer Verbindung der Formel (III):
A-OCOY (III)
worin Y die gleiche Bedeutung, wie oben gegeben, besitzt und A ein Wasserstoffatom oder ein Alkalimetall bedeutet,
oder den pharmazeutisch annehmbaren Salzen davon unter Bildung einer Verbindung der Formel (I) und, wenn erforderlich, Umwandlung der Verbindung der Formel (I) in ein pharmazeutisch annehmbares Salz davon in an sich bekannter Weise.

## Patentansprüche (Patentansprüche für folgende(n) Vertragsstaat(en): GR)

1. Verbindung der Formel (I): 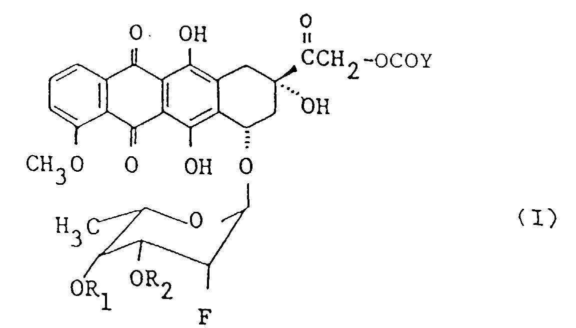 worin R₁ und R₂ ein Wasserstoffatom bedeuten oder zusammen eine geradkettige oder verzweigtkettige Alkylidengruppe mit 1 bis 10 Kohlenstoffatomen bilden,
Y 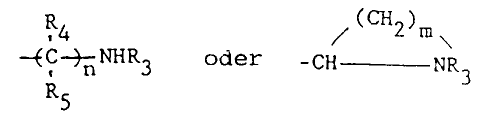 bedeutet,
R₃ ein Wasserstoffatom, eine C₁₋₁₀-Alkylqruppe oder eine C₁₋₁₀-Alkoxycarbonylgruppe bedeutet; R₄ und R₅ ein Wasserstoffatom oder eine C₁₋₅-Alkylgruppe bedeuten, n 0 oder eine ganze Zahl von 1 bis 10 bedeutet und m eine ganze Zahl von 1 bis 5 bedeutet,
und die Salze davon.

2. Verfahren zur Herstellung einer Verbindung der Formel (I): 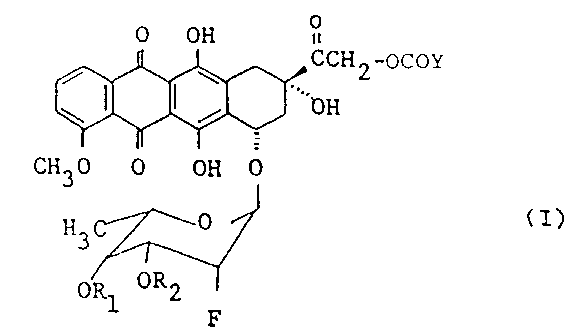 worin R₁ und R₂ ein Wasserstoffatom bedeuten oder zusammen eine geradkettige oder verzweigtkettige Alkylidengruppe mit 1 bis 10 Kohlenstoffatomen bilden,
Y 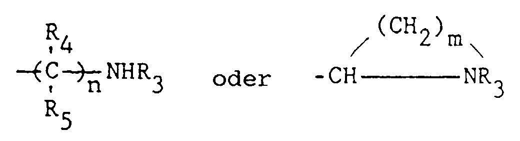 bedeutet,
R₃ ein Wasserstoffatom, eine C₁₋₁₀-Alkylgruppe oder eine C₁₋₁₀-Alkoxycarbonylgruppe bedeutet; R₄ und R₅ ein Wasserstoffatom oder eine C₁₋₅-Alkylgruppe bedeuten, n 0 oder eine ganze Zahl von 1 bis 10 bedeutet und m eine ganze Zahl von 1 bis 5 bedeutet,
und der Salze davon durch Umsetzung einer Verbindung der Formel (II): 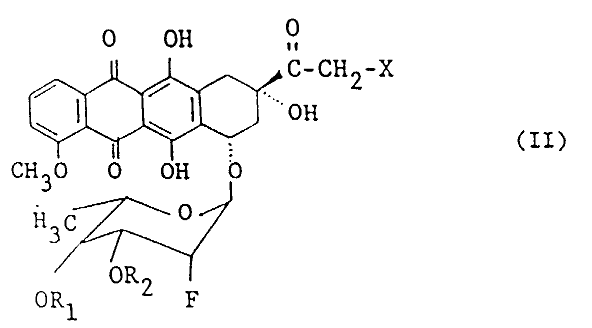 worin R₁ und R₂ die gleichen Bedeutungen, wie oben gegeben, besitzen und X ein Brom-, Chlor- oder Iodatom bedeutet,
mit einer Verbindung der Formel (III):
A-OCOY (III)
worin Y die gleiche Bedeutung, wie oben gegeben, besitzt und A ein Wasserstoffatom oder ein Alkalimetall bedeutet,
oder den Salzen davon unter Bildung einer Verbindung der Formel (I) und, wenn erforderlich, Umwandlung der Verbindung der Formel (I) in ein Salz davon in an sich bekannter Weise.

## Revendications (Revendications pour l'(les) Etat(s) contractant(s) suivant(s): AT, BE, CH, DE, DK, FR, GB, IT, LI, LU, NL, SE)

1. Composé de formule (I): 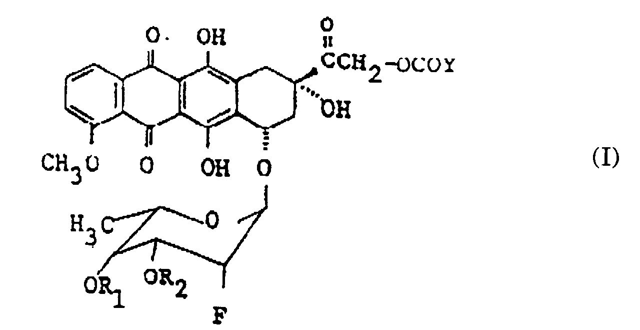 dans laquelle :
R₁ et R₂ représentent respectivement un atome d'hydrogène ou forment ensemble un groupe alkylidène à chaîne droite ou ramifiée de 1 à 10 atomes de carbone;
Y représente un groupe 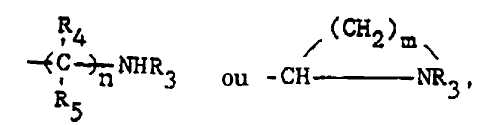
R₃ représente un atome d'hydrogène, un groupe alkyle en C₁₋₁₀ ou alcoxycarbonyle en C₁₋₁₀, R₄ et R₅ représentent respectivement un atome d'hydrogène ou un groupe alkyle en C₁₋₅, n représente 0 ou un entier de 1 à 10, et m représente un entier de 1 à 5,
et sel de celui-ci acceptable du point de vue pharmaceutique.

2. Procédé pour la préparation d'un composé de formule (I): 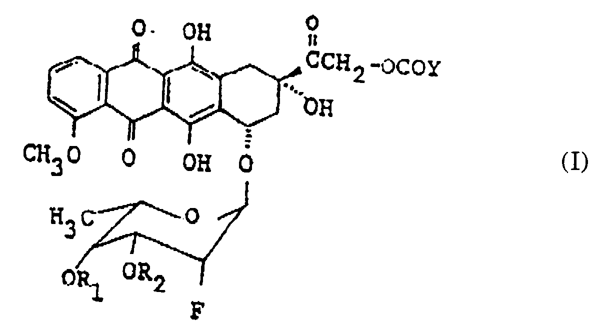 dans laquelle :
R₁ et R₂ représentent respectivement un atome d'hydrogène ou forment ensemble un groupe alkylidène à chaîne droite ou ramifiée de 1 à 10 atomes de carbone;
Y représente un groupe 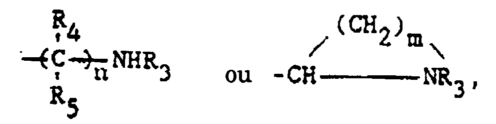
R₃ représente un atome d'hydrogène, un groupe alkyle en C₁₋₁₀ ou alcoxycarbonyle en C₁₋₁₀, R₄ et R₅ représentent respectivement un atome d'hydrogène ou un groupe alkyle en C₁₋₅, n représente 0 ou un entier de 1 à 10, et m représente un entier de 1 à 5,
et de sel de celui-ci acceptable du point de vue pharmaceutique,
par réaction d'un composé de formule (II): 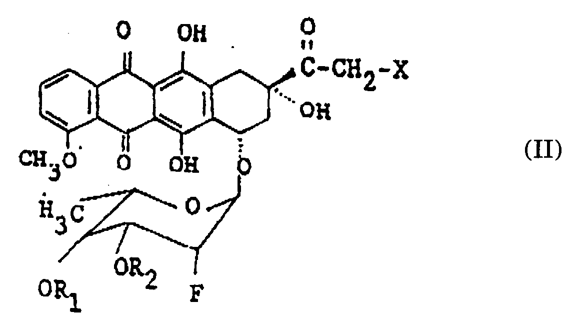 dans laquelle :
R₁ et R₂ sont tels que définis plus haut; et
X représente un atome de brome, de chlore ou d'iode,
avec un composé de formule (III) :
A-OCOY (III)
dans laquelle Y est tel que défini plus haut, et A représente un atome d'hydrogène ou un métal alcalin,
ou un sel de celui-ci acceptable du point de vue pharmaceutique, pour obtenir le composé de formule (I) et si cela est nécessaire, le composé de formule (I) est converti en sel de celui-ci acceptable du point de vue pharmaceutique par un moyen classique.

## Revendications (Revendications pour l'(les) Etat(s) contractant(s) suivant(s): ES)

1. Procédé pour la préparation d'un composé de formule (I): 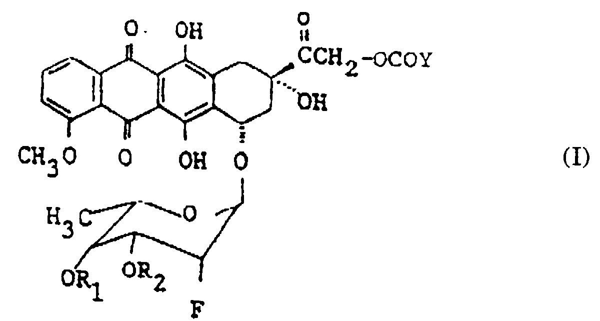 dans laquelle :
R₁ et R₂ représentent respectivement un atome d'hydrogène ou forment ensemble un groupe alkylidène à chaîne droite ou ramifiée de 1 à 10 atomes de carbone;
Y représente un groupe 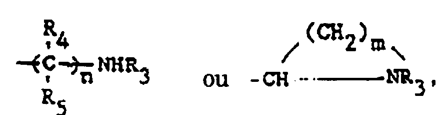
R₃ représente un atome d'hydrogène, un groupe alkyle en C₁₋₁₀ ou alcoxycarbonyle en C₁₋₁₀, R₄ et R₅ représentent respectivement un atome d'hydrogène ou un groupe alkyle en C₁₋₅, n représente 0 ou un entier de 1 à 10, et m représente un entier de 1 à 5,
et d'un sel de celui-ci acceptable du point de vue pharmaceutique,
par réaction d'un composé de formule (II): 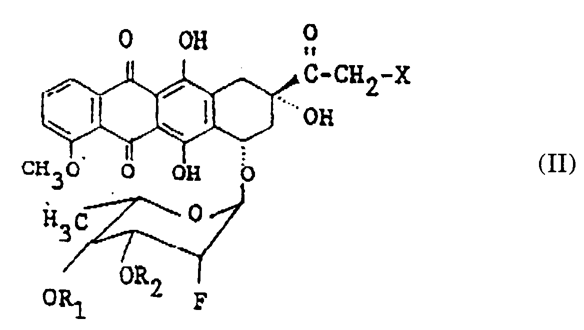 dans laquelle :
R₁ et R₂ sont tels que définis plus haut; et
X représente un atome de brome, de chlore ou d'iode,
avec un composé de formule (III) :
A-OCOY (III)
dans laquelle Y est tel que défini plus haut, et A représente un atome d'hydrogéne ou un métal alcalin,
ou un sel de celui-ci acceptable du point de vue pharmaceutique, pour obtenir le composé de formule (I) et si cela est nécessaire, le composé de formule (I) est converti en sel de celui-ci acceptable du point de vue pharmaceutique par un moyen classique.

## Revendications (Revendications pour l'(les) Etat(s) contractant(s) suivant(s): GR)

1. Composé de formule (I): 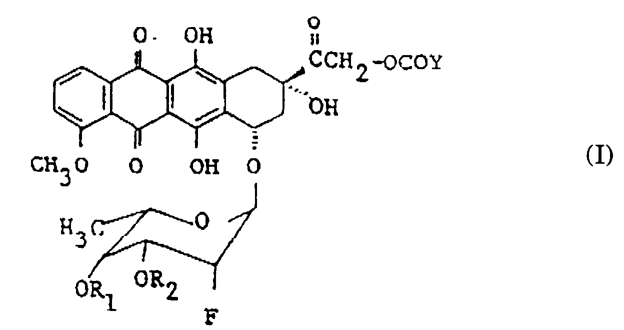 dans laquelle :
R₁ et R₂ représentent respectivement un atome d'hydrogène ou forment ensemble un groupe alkylidène à chaîne droite ou ramifiée de 1 à 10 atomes de carbone;
Y représente un groupe 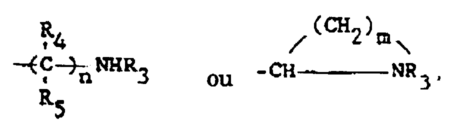
R₃ représente un atome d'hydrogène, un groupe alkyle en C₁₋₁₀ ou alcoxycarbonyle en C₁₋₁₀, R₄ et R₅ représentent respectivement un atome d'hydrogène ou un groupe alkyle en C₁₋₅, n représente 0 ou un entier de 1 à 10, et m représente un entier de 1 à 5,
et sel de celui-ci.

2. Procédé pour la préparation d'un composé de formule (I): 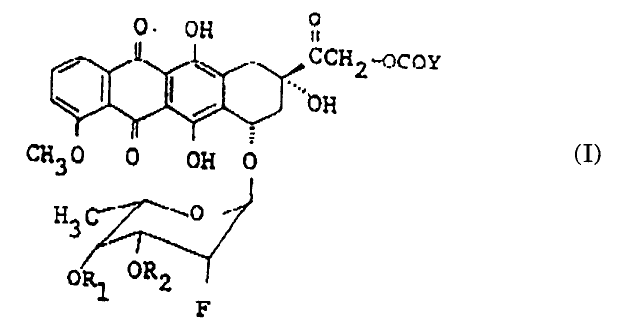 dans laquelle:
R₁ et R₂ représentent respectivement un atome d'hydrogène ou forment ensemble un groupe alkylidène à chaîne droite ou ramifiée de 1 à 10 atomes de carbone;
Y représente un groupe 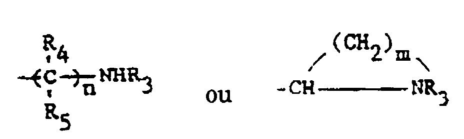
R₃ représente un atome d'hydrogène, un groupe alkyle en C₁₋₁₀ ou alcoxycarbonyle en C₁₋₁₀, R₄ et R₅ représentent respectivement un atome d'hydrogène ou un groupe alkyle en C₁₋₅, n représente 0 ou un entier de 1 à 10, et m représente un entier de 1 à 5,
et d'un sel de celui-ci,
par réaction d'un composé de formule (II): 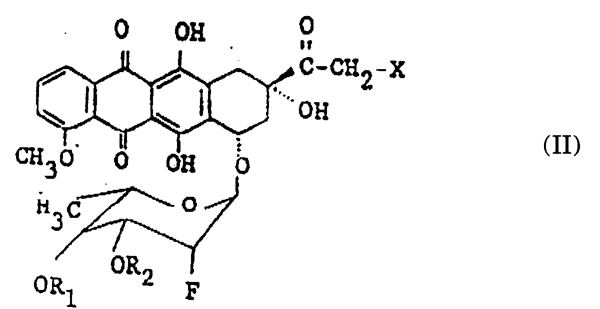 dans laquelle :
R₁ et R₂ sont tels que définis plus haut; et
X représente un atome de brome, de chlore ou d'iode,
avec un composé de formule (III) :
A-OCOY (III)
dans laquelle Y est tel que défini plus haut, et A représente un atome d'hydrogène ou un métal alcalin,
ou un sel de celui-ci ,pour obtenir le composé de formule (I) et si cela est nécessaire, le composé de formule (I) est converti en sel de celui-ci par un moyen classique.
